# DEMANDE DE BREVET EUROPEEN

(11) **EP 3 143 953 A1**
(43) Date de publication de la demande: **22.03.2017**
(21) Numéro de dépôt: 16189123.9
(22) Date de dépôt: 16.09.2016
(51) Int. Cl.: A61B 17/3217, A61B 17/00, A61B 17/30, A61B 17/3213

(54) **INSTRUMENT CHIRURGICAL DESTINE A RECEVOIR DE MANIERE AMOVIBLE UN OUTIL COUPANT, KIT CHIRURGICAL ET PROCEDE DE FABRICATION AFFERENTS**

(30) Priorité: 17.09.2015 FR 1558777
(71) Demandeur: In2Bones, 69130 Ecully (FR)
(72) Inventeur: BAERTICH, Christian, 31300 Toulouse (FR); VERNOIS, Joel, 90310 Picquigny (FR); RAY, Adrien, 1227 Carouge (CH); REDFERN, David Jonathan, Hove, BN33PA (GB)
(74) Mandataire: Weber, Jean-François

(57) **Abrégé**

- Instrument chirurgical destiné à recevoir de manière amovible un outil coupant, kit chirurgical et procédé de fabrication afférents.
- L'invention concerne un instrument chirurgical (1) destiné à recevoir de manière amovible un outil coupant comportant :
- un organe de réception (7), comportant un corps (8) doté d'un moyen de réception (10) de l'outil, lequel est conçu pour évoluer entre une configuration verrouillée et une configuration déverrouillée,
-un dispositif de verrouillage / déverrouillage (14) comprenant un premier élément (15) relié à l'organe de réception (7) par liaison hélicoïdale,
ledit instrument chirurgical (1) étant caractérisé en que ledit dispositif de verrouillage / déverrouillage (14) comprend un deuxième élément (22) relié à l'organe de réception (7) par liaison glissière, pour qu'une rotation relative des premier (15) et deuxième (22) éléments entraîne une translation relative de l'organe de réception (7) et du deuxième élément (22) permettant de commander le moyen de réception (10).
- Instruments chirurgicaux destinés à recevoir de manière amovible un outil coupant.

## Description

La présente invention concerne le domaine de l'instrumentation chirurgicale et en particulier des outils chirurgicaux destinés à recevoir de manière amovible un outil coupant, par exemple une lame de bistouri, afin de permettre au chirurgien de changer ou de choisir facilement et rapidement l'outil avant ou pendant une opération chirurgicale de sorte à réaliser au mieux ladite opération.

La présente invention concerne plus particulièrement un instrument chirurgical destiné à recevoir de manière amovible un outil coupant, par exemple une lame de bistouri, camportant :
- un organe de réception, qui comporte lui-même un corps principal primaire allongé avec une extrémité distale primaire, laquelle est dotée d'un moyen de réception de l'outil coupant, ledit moyen de réception étant conçu pour évoluer entre une configuration verrouillée et une configuration déverrouillée,
- un dispositif de verrouillage / déverrouillage, qui permet de faire passer le moyen de réception de sa configuration verrouillée à sa configuration déverrouillée et vice versa, ledit dispositif de verrouillage / déverrouillage comprenant un premier élément relié à l'organe de réception par une liaison hélicoïdale et un deuxième élément, comprenant un corps principal secondaire présentant une paroi extérieure, relié à l'organe de réception par une liaison glissière, pour qu'une rotation relative des premier et deuxième éléments entraîne une translation relative de l'organe de réception et du deuxième élément permettant ainsi de commander le verrouillage / déverrouillage du moyen de réception.

La présente invention concerne également un procédé de fabrication d'un tel instrument chirurgical.

La présente invention concerne en outre un kit chirurgicale destiné à la mise en oeuvre d'une technique opératoire, préférentiellement percutanée, notamment dans le cadre du traitement d'une pathologie du pied d'un patient, comprenant un tel instrument chirurgical.

Dans le domaine des outils chirurgicaux destinés à recevoir de manière amovible un outil coupant, par exemple une lame de bistouri, on connaît des outils de type « *manche de bistouri »* composés d'un manche allongé cylindrique, généralement réalisé en métal et réutilisable, et d'un mandrin serrant apte à recevoir, de manière amovible, des lames de bistouri, à usage unique ou non, et de différentes formes.

Le mandrin, de forme sensiblement cylindrique comporte en l'une de ses extrémités une tête conique pourvue d'une ou plusieurs fentes axiales, formant des éléments de mâchoire, destinées à recevoir le talon de la lame, c'est-à-dire son extrémité non coupante. En son autre extrémité, le mandrin est doté d'un filetage externe. Ce mandrin est monté coulissement dans un trou axial longitudinal ménagé dans le manche et au fond duquel est prévu un taraudage.

Une fois le talon d'une lame de bistouri insérée dans une fente du mandrin, on vient faire coulisser puis visser ce dernier à l'intérieur du manche, jusqu'à ce que l'appui de la tête du mandrin vienne coopérer avec le bord du trou axial, de sorte à provoquer le resserrement des éléments de mâchoire du mandrin sur le talon de la lame et de solidariser ainsi la lame amovible au manche pour former un bistouri.

Si de tels instruments connus donnent généralement satisfaction dans le cadre de la mise en oeuvre de techniques chirurgicales « à *ciel ouvert* », c'est-à-dire des techniques impliquant la pratique d'une incision large et ouverte de la peau et des tissus mous, la lame de bistouri restant le plus souvent visible à l'extrémité du manche, leur utilisation est sensiblement plus délicate dans le cadre de la mise en oeuvre d'une technique opératoire percutanée, par exemple dans le cadre du traitement de pathologies osseuses du pied par voie percutanée.

En effet, le chirurgien perd alors tout contact visuel avec la lame lorsque celle-ci est intégralement insérée dans le corps du patient à travers une très fine incision pratiquée dans la peau et les tissus mous. En particulier, sauf à retirer ponctuellement l'extrémité du bistouri du corps du patient, le chirurgien n'a plus accès à l'information quant à l'orientation de la lame relativement au manche porte-lame, ce qui peut se révéler très problématique, le chirurgien risquant alors de couper involontairement des tissus mous environnants.

L'invention vise en conséquence à porter remède à cet inconvénient majeur, et à proposer un instrument chirurgical, destiné à recevoir de manière amovible un outil coupant, plus ergonomique et plus sûr, particulièrement bien adapté à une utilisation dans le cadre d'une technique opératoire percutanée.

Un autre objet de l'invention vise à proposer un nouvel instrument chirurgical, destiné à recevoir de manière amovible un outil coupant, particulièrement sûr à utiliser.

Un autre objet de l'invention vise à proposer un nouveau procédé de fabrication d'un instrument chirurgical à la fois simple et peu coûteux à mettre en oeuvre.

Un autre objet de l'invention vise à proposer un nouvel instrument chirurgical, destiné à recevoir de manière amovible un outil coupant, relativement peu coûteux à fabriquer.

Un autre objet de l'invention vise à proposer un nouveau kit chirurgical destiné à la mise en oeuvre d'une technique opératoire, préférentiellement percutanée, complet et ergonomique.

Un autre objet de l'invention vise à proposer un nouveau kit chirurgical destiné à la mise en oeuvre d'une technique opératoire, préférentiellement percutanée, sûr et peu coûteux.

Les objets assignés à l'invention sont atteints à l'aide d'un instrument chirurgical destiné à recevoir de manière amovible un outil coupant, par exemple une lame de bistouri, comportant :
- un organe de réception, qui comporte lui-même un corps principal primaire allongé avec une extrémité distale primaire, laquelle est dotée d'un moyen de réception de l'outil coupant, ledit moyen de réception étant conçu pour évoluer entre une configuration verrouillée et une configuration déverrouillée,
- un dispositif de verrouillage / déverrouillage, qui permet de faire passer le moyen de réception de sa configuration verrouillée à sa configuration déverrouillée et vice versa, ledit dispositif de verrouillage / déverrouillage comprenant un premier élément relié à l'organe de réception par une liaison hélicoïdale, et un deuxième élément, comprenant un corps principal secondaire présentant une paroi extérieure, relié à l'organe de réception par une liaison glissière, pour qu'une rotation relative des premier et deuxième éléments entraîne une translation relative de l'organe de réception et du deuxième élément permettant ainsi de commander le verrouillage / déverrouillage du moyen de réception,
caractérisé en ce que ledit premier élément constitue un manche de préhension dudit instrument chirurgical et en ce que le dispositif de verrouillage / déverrouillage est pourvu d'un repère qui reflète la position de l'outil coupant relativement au deuxième élément, ledit repère étant positionné axialement sur ladite paroi extérieure.

Les objets assignés à l'invention sont également atteints à l'aide d'un procédé de fabrication d'un tel instrument chirurgical, caractérisé en ce qu'on réalise par moulage ledit instrument chirurgical dans son intégralité.

Les objets assignés à l'invention sont également atteints à l'aide d'un kit chirurgical destiné à la mise en oeuvre d'une technique opératoire, préférentiellement percutanée, notamment dans le cadre du traitement d'une pathologie du pied d'un patient, comprenant un tel instrument chirurgical, et étant caractérisé en ce qui comprend également au moins un écarteur, une rugine et une râpe.

D'autres objets et avantages de l'invention apparaîtront plus en détail à la lecture de la description qui suit, ainsi qu'à l'aide des dessins annexés fournis à titre purement explicatif et non limitatif, dans lesquels :
- La figure 1 illustre, en perspective, un instrument chirurgical destiné à recevoir de manière amovible un outil coupant conforme à l'invention ;
- La figure 2 illustre, en vue de profil, un exemple d'outil coupant, sous la forme d'une lame de bistouri, apte à coopérer avec l'instrument de l'invention ;
- La figure 3 illustre, en perspective, l'organe de réception de l'instrument chirurgical de la figure 1 ;
- La figure 4 illustre, en transparence, le mode de réalisation préférentiel particulier du taraudage, sous la forme de deux demi-taraudages, du premier élément relié à l'organe de réception de l'instrument de la figure 1 par une liaison hélicoïdale ;
- La figure 5 illustre, en perspective, un mode de réalisation préférentiel de l'organe de réception et du deuxième élément du dispositif de verrouillage / déverrouillage auquel il est relié par une liaison glissière ;
- La figure 6 illustre, en coupe, le mode de réalisation préférentiel de la figure 5, de sorte à bien faire apparaître le dispositif de blocage qui permet de réaliser cette liaison glissière ;
- La figure 7 illustre, en vue de dessus, un mode de réalisation préférentiel d'un kit chirurgical conforme à l'invention ;
- La figure 8 illustre en vue de profil un mode de réalisation d'une râpe telle que préférentiellement incluse dans le kit chirurgical de la figure 7 ;
- Les figures 9 à 12 illustrent différentes configurations géométriques que peuvent présenter les dents de la râpe de la figure 8.

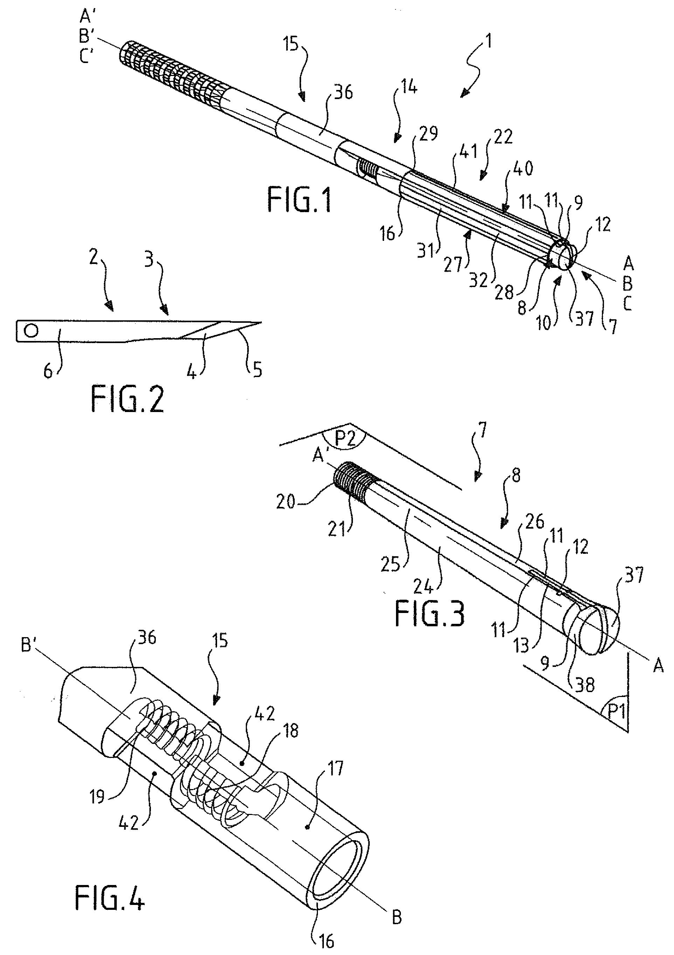

L'invention concerne un instrument chirurgical 1 destiné à recevoir de manière amovible un outil coupant, par exemple une lame 2 de bistouri, dont un exemple de réalisation conforme à l'invention est illustré à la figure 1. En l'espèce, l'instrument chirurgical 1 de l'invention forme un manche porte-lame, ou « *beaver »* (par lexicalisation de la marque Beaver®), particulièrement adapté pour une utilisation par un chirurgien au cours d'une opération chirurgicale percutanée sur un patient, par exemple dans le cadre du traitement d'une pathologie du pied, et de manière plus générale, pour le traitement par voie percutanée de pathologies simples ou facilement accessibles (fractures simples ou peu déplacées, ruptures tendineuses, lésions diverses bien délimitées, etc.). Bien entendu, sans sortir du cadre de l'invention, l'instrument chirurgical 1 pourra être utilisé dans le cadre d'une technique opératoire non percutanée, sa mise en oeuvre se révélant particulièrement avantageuse dès lors que se pose une problématique de visualisation et de maîtrise précise de l'orientation de l'objet coupant qui lui est rapporté. Non restreint à un usage exclusif à un patient humain, l'instrument chirurgical 1 pourra également être mise en oeuvre dans le cadre d'une chirurgie animale, par voie percutanée ou non.

Dans ce qui suit, l'exemple sera pris d'un objet coupant se présentant sous la forme d'une lame 2 de bistouri, telle qu'illustrée à la figure 2. Une telle lame 2 comporte un corps plat 3 comprenant une tête de coupe 4, dotée d'au moins un bord coupant 5, prolongée par un talon 6 rectangulaire. Il est bien évidemment parfaitement possible, sans sortir du cadre de l'invention, que d'un objet coupant en question se présente autrement que sous la forme d'une lame 2 de bistouri, par exemple sous la forme d'une lame en dents de scie. De préférence, et tel qu'illustré à la figure 2, l'outil coupant est donc un outil sensiblement plat et allongé, et l'instrument 1 est avantageusement conçu, comme cela va être décrit ci-après, pour le recevoir dans une configuration *« droite »,* c'est-à-dire de manière à ce que l'outil coupant, lorsqu'il est assemblé à l'instrument, s'étende selon le même axe d'extension moyen que ce dernier.

Selon une caractéristique importante de l'invention, l'instrument chirurgical 1 comporte un organe de réception 7, qui comporte lui-même un corps principal primaire 8 allongé, s'étendant préférentiellement selon un premier axe longitudinal A-A', avec une extrémité distale primaire 9, laquelle est dotée d'un moyen de réception 10 de l'outil coupant, par exemple une lame 2 de bistouri. Ledit outil coupant vient avantageusement interagir avec le moyen de réception 10 par l'intermédiaire d'une de ses extrémités non coupante, typiquement, dans le cas d'une lame 2 de bistouri, par l'intermédiaire du talon 6 de ladite lame 2. Ledit moyen de réception 10 est conçu pour évoluer entre une configuration verrouillée et une configuration déverrouillée, c'est-à-dire entre respectivement une configuration dans laquelle l'outil coupant est fermement maintenu en position par ledit moyen de réception 10 et une configuration dans laquelle, au contraire, l'outil coupant est libre et peut être désolidarisé du moyen de réception 10.

De manière préférentielle, le moyen de réception 10 comprend au moins deux éléments de réception 11 flexibles définissant une fente 12 axiale allongée apte à recevoir ledit outil coupant. Cette fente 12, traversante, s'étendant avantageusement selon un premier plan d'extension P1 dans lequel est inclus ledit premier axe longitudinal A-A', et ce depuis ladite extrémité distale primaire 9 et sur au moins une portion de la longueur du corps principal primaire 8 de l'organe de réception 7. Comme illustré aux figures, lesdits éléments de réception 11 forment ainsi de façon avantageuse des éléments de mâchoire aptes à évoluer élastiquement entre une position de rappel élastique dans laquelle les faces internes 13 de ladite fente 12 qu'ils définissent tendent naturellement à rester parallèles et écartées l'une de l'autre, et une position de contrainte dans laquelle les éléments de réception 11 sont au contraire fléchis de sorte que lesdites faces internes 13 tendent à converger l'une vers l'autre. Ainsi, lorsque le moyen de réception 10 est dans configuration verrouillée, les éléments de réception 11 du moyen de réception 10 adoptent leur position de contrainte et viennent immobiliser, par compression entre lesdites faces internes 13 des éléments de réception, la partie de l'outil coupant placée entre dans ladite fente 12.

Selon une caractéristique importante de l'invention, l'instrument chirurgical 1 comporte un dispositif de verrouillage / déverrouillage 14, qui permet de faire passer le moyen de réception 10 de sa configuration verrouillée à sa configuration déverrouillée et vice versa. Ce dispositif de verrouillage / déverrouillage 14 permet avantageusement de sécuriser de manière réversible la réception de l'outil coupant par le moyen de réception 10 de l'organe de réception 7 en forçant les éléments de réception 11 à adopter leur position de contrainte pour éviter qu'ils ne reprennent inopinément leur position de rappel élastique et ne libèrent l'outil coupant.

Ledit dispositif de verrouillage / déverrouillage 14 comprend un premier élément 15, qui s'étend préférentiellement selon un deuxième axe longitudinal B-B', relié à l'organe de réception 7 par une liaison hélicoïdale, autrement dit par une liaison de type vis / écrou. Pour réaliser cette liaison hélicoïdale, ce premier élément 15 comporte de préférence une extrémité de couplage 16 pourvue d'un trou axial taraudé 17 dont le taraudage est constitué de deux demi-taraudages 18, 19. En d'autres termes, et tel qu'illustré à la figure 4, le taraudage du trou axial taraudé 17 n'est pas constitué d'un seul taraudage circulaire complet classique mais, de préférence, de la juxtaposition de deux demi-taraudages 18, 19, ces derniers étant positionnés de manière décalée l'un par rapport à l'autre selon le deuxième axe longitudinal B-B', de manière opposée de part et d'autre du deuxième axe longitudinal B-B', et de sorte que la fin du premier demi-taraudage 18 coïncide avec le début du deuxième demi-taraudage 19. De son côté, l'organe de réception 7 est préférentiellement prolongé axialement à partir d'une extrémité proximale primaire 20 par une barre filetée 21. Celle-ci est avantageusement conçue de sorte à coopérer avec les demi-taraudages 18, 20 dudit trou axial taraudé 17, les premier A-A' et deuxième B-B' axes longitudinaux étant alors préférentiellement confondus. Ainsi, lorsque l'outil coupant forme un outil droit, non recourbé ni angulé, ce dernier peut être maintenu de manière droite, c'est-à-dire de sorte que son axe d'extension soit confondu avec les premier A-A' et deuxième B-B' axes longitudinaux.

Selon l'invention, et comme cela va être progressivement décrit et explicité en détail ci-après, le dispositif de verrouillage / déverrouillage 14 comprend un deuxième élément 22 relié à l'organe de réception 7 par une liaison glissière, pour qu'une rotation relative des premier 15 et deuxième 22 éléments entraîne une translation relative de l'organe de réception 7 et du deuxième élément 22 permettant ainsi de commander le verrouillage / déverrouillage du moyen de réception 10. De façon préférentielle, cette liaison glissière est réalisée à partir d'une liaison pivot glissant relativement à l'axe A-A' dont la rotation autour dudit axe A-A' est interdite par au moins un dispositif de blocage 23.

Selon le mode de réalisation préférentiel illustré notamment à la figure 3, ledit corps principal primaire 8 de l'organe de réception 7 est de forme sensiblement cylindrique, matérialisant avantageusement une tige 24, et présente une surface extérieure 25, qui relie avantageusement ladite extrémité distale primaire 9 à ladite extrémité proximale primaire 20. Ladite surface extérieure 25 est pourvue d'au moins un méplat primaire 26 axial s'étendant :
- longitudinalement depuis ladite extrémité distale primaire 9 sur au moins une portion de la longueur dudit corps principal primaire 8 de l'organe de réception 7,
- et selon un deuxième plan d'extension P2 orthogonal au premier plan d'extension P1 de ladite fente 22.

Ce méplat primaire 26 forme une surface plane dans la surface extérieure 25, caractérisée par une suppression ou une absence de matière, et qui s'étend préférentiellement selon un plan orthogonal au premier plan d'extension P1 de ladite fente 12. De manière préférentielle, il s'étend selon le premier axe longitudinal A-A' jusqu'à ladite extrémité proximale primaire 20 du corps principal primaire 8.

De façon préférentielle, ledit deuxième élément 22 comprend un corps principal secondaire 27, s'étendant entre une extrémité distale secondaire 28 et une extrémité proximale secondaire 29, pourvu d'un trou axial cylindrique 30 traversant, apte à recevoir étroitement, c'est-à-dire sensiblement sans jeu, ledit corps principal primaire 8 de l'organe de réception 7. Ledit corps principal secondaire 27 s'étend donc de préférence selon un troisième axe longitudinal C-C' confondu avec le premier axe longitudinal A-A' lorsque ledit corps principal primaire 8 est monté dans le trou axial cylindrique 30 traversant dudit deuxième élément 22. Selon le mode de réalisation préférentiel illustré aux figures, ce corps principal secondaire 27 est de forme sensiblement cylindrique et forme ainsi un manchon 31 à l'intérieur duquel le corps principal primaire 8 de l'organe de réception 7 peut évoluer. La paroi extérieure 32 du corps principal secondaire 27, qui relie préférentiellement lesdites extrémité distale secondaire 28 et extrémité proximale secondaire 29, peut être avantageusement légèrement cannelée afin de garantir au chirurgien une meilleure préhension manuelle du deuxième élément 22 du dispositif de verrouillage / déverrouillage 14 malgré l'usage de gants médicaux. De manière alternative, il est tout à fait concevable à ce titre, sans sortir du cadre de l'invention, que le corps principal secondaire 27 du deuxième élément 22 se présente par exemple sous la forme générale d'un prisme droit à base triangulaire, carrée ou encore hexagonale, pourvu d'un trou axial cylindrique 30 traversant.

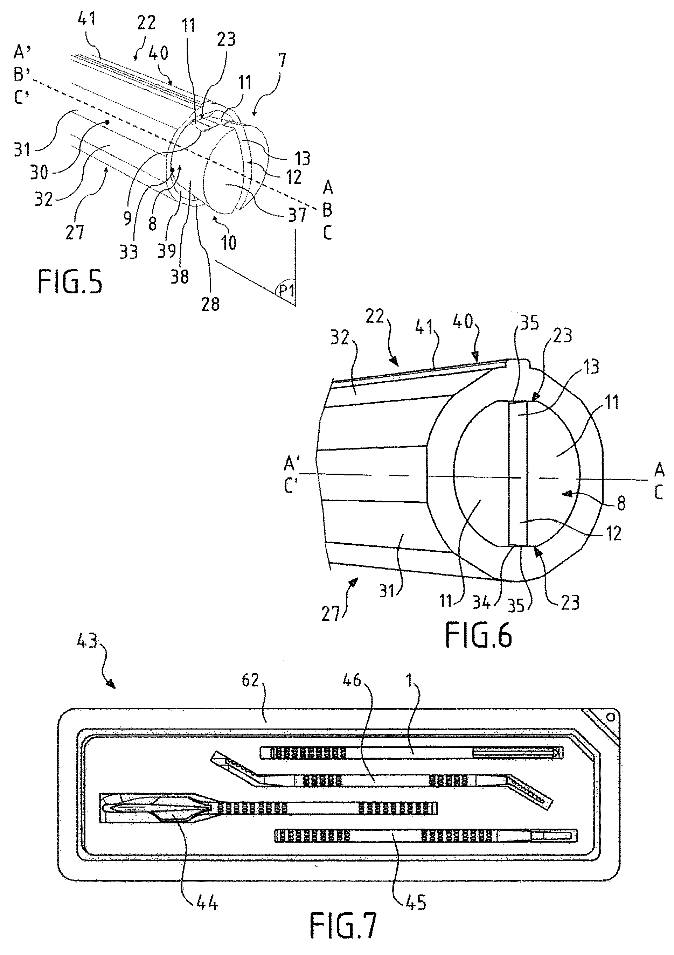

Ledit trou axial cylindrique 30 débouche préférentiellement de ladite extrémité distale secondaire 28 à travers un orifice distal et de ladite extrémité proximale secondaire 29 à travers un orifice proximal (non représenté). Le corps principal primaire 8 de l'organe de réception 7 est ainsi monté amovible relativement au deuxième élément 22 et peut évoluer dans le trou axial cylindrique 30 du corps principal secondaire 27 du deuxième élément 22 entre lesdits orifices distal 33 et proximal. Tel qu'illustré en particulier à la figure 5, ledit trou axial cylindrique 30 définit une paroi intérieure 34, laquelle est avantageusement pourvue d'au moins un méplat secondaire 35 axial, s'étendant de préférence longitudinalement depuis ladite extrémité distale secondaire 28 sur au moins une portion de la longueur dudit corps principal secondaire 27 du deuxième élément 22, de sorte que ce méplat secondaire 35 forme par coopération avec le méplat primaire 26 un dispositif de blocage 23 apte à interdire toute rotation d'axe A-A' dudit organe de réception 7 dans ledit deuxième élément 22 du dispositif de verrouillage / déverrouillage 14.

Ledit corps principal primaire 8 de l'organe de réception 7 étant préférentiellement monté dans le trou axial cylindrique 30 dudit corps principal secondaire 27 du deuxième élément 22, de manière à ce que les premier A-A' et troisième C-C' axes longitudinaux soient confondus et à ce que lesdits au moins un méplat primaires 26 et secondaire 35 axiaux soient positionnés en regard l'un de l'autre, comme illustré notamment à la figure 5, la liaison pivot glissant formée par ledit corps principal primaire 8 de l'organe de réception 7 et ledit corps principal secondaire 27 du deuxième élément 22 est avantageusement transformée en une liaison glissière. La translation selon le premier axe A-A' du corps principal primaire 8 de l'organe de réception 7 dans le trou axial cylindrique 30 dudit corps principal secondaire 27 du deuxième élément 22 est en effet autorisée, tandis que la rotation dudit corps principal primaire 8 autour de ce premier axe A-A' est de fait interdite par la coopération desdits méplats primaire 26 et secondaire 35.

De manière particulièrement avantageuse, et comme cela est particulièrement visible à la figure 5, ladite surface extérieure 25 du corps principal primaire 8 de l'organe de réception 7 est pourvue de deux méplats primaires 26 axiaux, et ladite paroi intérieure 34 est pourvue de deux méplats secondaires 35 axiaux.

Lesdits méplats primaires 26 axiaux et méplats secondaires 35 axiaux sont respectivement positionnés en opposition radiale l'un par rapport à l'autre, de sorte lesdits méplats secondaires 35 forment respectivement par coopération avec lesdits méplats primaires 26 un double un dispositif de blocage 23 apte à interdire toute rotation d'axe A-A' dudit organe de réception 7 dans ledit deuxième élément 22 du dispositif de verrouillage / déverrouillage 14.

Cette variante préférentielle à deux jeux de méplats primaires 26 et secondaires 35 permet avantageusement de garantir un blocage parfait de la rotation du corps principal primaire 8 de l'organe de réception 7 dans le trou axial cylindrique 30 dudit corps principal secondaire 27 du deuxième élément 22.

Tel que décrit ci-dessus, le deuxième élément 22 du dispositif de verrouillage / déverrouillage 27 est donc relié à l'organe de réception 7 par une liaison glissière pure. Cette dernière est avantageusement configurée de telle sorte que, lorsque la barre filetée 21 du corps principal primaire 8 de l'organe de réception 7 vient coopérer avec le taraudage du trou axial taraudé 17 du premier élément 15 afin de relier ce dernier à l'organe de réception 7 par une liaison hélicoïdale, une rotation relative des premier 15 et deuxième 22 éléments entraîne une translation pure selon le premier axe longitudinal A-A' de l'organe de réception 7 dans le deuxième élément 22.

En outre, lorsque l'organe de réception 7 est monté dans le deuxième élément 22 du dispositif de verrouillage / déverrouillage 14, la position angulaire dudit deuxième élément 22 relativement au premier axe longitudinal A-A' correspond en permanence parfaitement à la position angulaire de l'organe de réception 7 relativement à cet premier axe A-A'.

De manière alternative, il est tout à fait envisageable, sans sortir du cadre de l'invention, que cette liaison glissière pure soit au contraire réalisée à partir d'un appui plan auquel on adjoint un second plan de contact sécant avec le premier ou, plus simplement, un axe de guidage latéral parallèle au plan.

Une telle liaison pourrait alors, par exemple être réalisée à l'aide d'un corps principal primaire 8 de section carrée ou hexagonale coulissant dans un deuxième élément 22 pourvue d'un trou axial 30 de section respectivement carrée ou hexagonale.

Selon le mode de réalisation préférentiel illustré à la figure 1, ledit premier élément 15 du dispositif de verrouillage / déverrouillage 14 constitue avantageusement un manche de préhension 36 dudit instrument chirurgical 1. En effet, dans ce mode de réalisation, ledit premier élément 15 s'étend longitudinalement sur une longueur sensiblement plus grande, par exemple plus de deux fois plus grande, que le deuxième élément 22 du dispositif de verrouillage / déverrouillage 14, de sorte à former de manière pratique ledit manche de préhension 36.

Toutefois, il est parfaitement envisageable de concevoir un mode de réalisation alternatif, dans lequel ledit premier élément 15 serait au contraire sensiblement plus court que ledit deuxième élément 22. Dans ce cas, ledit deuxième élément 22 formerait alors avantageusement le manche de préhension 36, et la configuration de l'organe de réception 7 serait modifiée en conséquence, sans pour autant impacter les caractéristiques et fonctionnalités techniques de l'instrument chirurgical 1 ni altérer son ergonomie.

De façon préférentielle, l'organe de réception 7 comprend une tête 37, visible aux figures 1 et 3, plus large que ledit corps principal primaire 8. Cette tête 37 prolonge axialement, c'est-à-dire préférentiellement selon le premier axe longitudinal A-A', ce dernier à partir de ladite extrémité distale primaire 9, et est formée par l'évasement dudit premier corps principal 8. En d'autres termes, ladite tête 37 est formée par l'élargissement progressif en direction de l'extrémité distale secondaire 9 de la section du corps principal primaire 8.

Dans la configuration préférentielle décrite ci-avant dans laquelle le corps principal primaire 8 de l'organe de réception 7 est de forme sensiblement cylindrique, ladite tête 37 présente ainsi une section circulaire de diamètre plus élevé que le diamètre de la section moyenne dudit corps principal primaire 8. Par ailleurs, le diamètre de la section circulaire de ladite tête 37 est avantageusement supérieur au diamètre de l'orifice distal 33 que présente l'extrémité distale secondaire 9 du corps principal secondaire 27 du deuxième élément 22.

Tel qu'illustré, ladite tête 37 fait avantageusement partie intégrante dudit moyen de réception 10, la fente 12 définie par les éléments de réception 11 se prolongeant axialement à travers ladite tête 37. De façon encore plus préférentielle, l'évasement dudit corps principal primaire 8 forme une surface d'appui 38 pentue de ladite tête 37 destinée à venir coopérer avec un bord intérieur 39, préférentiellement chanfreiné comme illustré à la figure 5, dudit orifice distal 33, de sorte à permettre l'immobilisation, par compression entre les éléments de réception 11, de l'outil coupant dans le moyen de réception 10.

Ainsi, lorsque la barre filetée 21 du corps principal primaire 8 de l'organe de réception 7 vient coopérer avec le taraudage du trou axial taraudé 17 du premier élément 15, une rotation relative des premier 15 et deuxième 22 éléments entraîne une translation, selon le premier axe longitudinal A-A', de l'organe de réception 7 dans le deuxième élément 22, jusqu'à ce que d'une part, l'extrémité proximale secondaire 29 du corps principal secondaire 27 du deuxième élément 22 vienne en contact avec l'extrémité de couplage 16 du premier élément 15 et que, d'autre part, la surface d'appui 38 de la tête 37 vienne coopérer avec le bord intérieur 39 de l'orifice distal 33 du deuxième élément 22 afin de faire évoluer les éléments de réception 11 du moyen de réception 10 de leur position de rappel élastique à leur position de contrainte dans laquelle ils sont fléchis de sorte que leurs faces internes 13 tendent à converger l'une vers l'autre. Corolairement, une rotation relative en sens inverse des premier 15 et deuxième 22 éléments entraîne une translation de l'organe de réception 7 dans le deuxième élément 22, de nature à faire cesser le contact entre, d'une part, les premier 15 et deuxième 22 éléments et, d'autre part, la surface d'appui 38 de la tête 37 et le bord intérieur 39 de l'orifice distal 33 du deuxième élément 22 afin de faire évoluer les éléments de réception 11 du moyen de réception 10 de leur position de contrainte à leur position de rappel élastique dans laquelle les faces internes 13 de ladite fente 12 qu'ils définissent tendent naturellement à rester parallèles et écartées l'une de l'autre. Cette translation de l'organe de réception 7 dans le deuxième élément 22 permet ainsi de commander respectivement le verrouillage et le déverrouillage du moyen de réception 10.

De façon préférentielle, le dispositif de verrouillage / déverrouillage 14 est pourvu d'un repère 40 de la position de l'outil coupant relativement à l'organe de réception 7 et également, de manière encore plus avantageuse, de la position de l'outil coupant relativement au deuxième élément 22. Ainsi, non seulement l'objet coupant est maintenu de manière ferme dans le moyen de réception 10 de l'organe de réception 7 de l'instrument chirurgical 1 dans une position déterminée relativement au deuxième élément 22 du dispositif de verrouillage / déverrouillage 14, mais de plus cette position déterminée est reflétée par ledit repère 40, de sorte à faciliter et à sécuriser l'utilisation de l'instrument chirurgical 1 par le chirurgien.

Ledit corps principal secondaire 27 du deuxième élément 22 présentant, comme introduit ci-avant, une paroi extérieure 32 qui relie préférentiellement lesdites extrémité distale secondaire 28 et extrémité proximale secondaire 29, ledit repère 40 est avantageusement positionné axialement, c'est-à-dire préférentiellement selon le troisième axe longitudinal C-C', sur ladite paroi extérieure 32.

De préférence, le repère 40 est positionné de sorte que, lorsque le deuxième élément 22 se trouve avantageusement positionné entre l'outil coupant et le premier élément 15, il soit plus proche de l'objet coupant que du premier élément qui constitue le manche de préhension de l'instrument 1. De façon encore préférentielle, ledit repère 40 s'étend longitudinalement à partir de ladite extrémité distale secondaire 28 sur au moins une partie, et préférentiellement sur la totalité, de la longueur du corps principal secondaire 27 dudit deuxième élément 22. Ainsi avantageusement positionné à proximité immédiate de l'outil coupant en place dans l'organe de réception 7, le repère 40 permet donc une identification aisée et rapide de l'orientation dudit outil coupant, puisque la rotation de l'organe de maintien 7 relativement au deuxième élément 22 lors du verrouillage de l'outil coupant est bloquée. L'instrument chirurgical 1 est dès lors particulièrement bien adapté à une mise en oeuvre dans le cadre d'une technique opératoire percutanée, le repère 40 restant avantageusement perceptible par le chirurgien même en cas d'opération profonde nécessitant l'introduction d'une partie du deuxième élément 22 dans le corps du patient à travers une fine incision pratiquée dans sa peau.

De manière particulièrement avantageuse, ledit repère 40 est positionné en regard d'un seul méplat secondaire 35. Autrement dit, le repère 40 constitue un unique élément de repère 40 positionné sur la paroi extérieure 32 du corps principal secondaire 27 du deuxième élément 22 du dispositif de verrouillage / déverrouillage 14, de sorte à suivre fidèlement sur toute ou partie de sa longueur ledit méplat secondaire 35 dont est pourvue la paroi intérieure 34 dudit corps principal secondaire 27. Dans le cas préférentiel où ladite paroi intérieure 34 est pourvue de deux méplats secondaires 35 axiaux, ledit repère 40 n'est donc ainsi positionné en regard que de l'un des deux méplats secondaires 35 axiaux. Dès lors, lorsque ledit méplat secondaire 35, en regard duquel est positionné le repère 40, coopère avec le, ou l'un des, méplat primaire 26 axial dont est pourvu le corps principal primaire 8 de l'organe de réception 7, lequel méplat primaire 26 s'étend selon ledit deuxième plan d'extension P2 orthogonal au premier plan d'extension P1 de ladite fente 12, le repère 40 apporte donc une information quant à l'orientation angulaire du premier plan d'extension P1 de ladite fente 12 par rapport aux premier A-A' et troisième C-C' axes longitudinaux.

Dans le cas pris en exemple où l'outil coupant est une lame 2 de bistouri, le talon 6 de cette dernière étant logé entre les éléments de réception 11 définissant ladite fente 12 et le moyen de réception 10 étant dans sa configuration verrouillée, le repère 40 permet donc d'identifier l'orientation angulaire de la lame 2 relativement aux premier A-A' et troisième C-C' axes longitudinaux. Même lorsqu'il n'a pas un contact visuel direct avec la lame 2, par exemple dans le cas d'une opération par voie percutanée, le chirurgien connaît ainsi cependant toujours l'orientation de sa lame 2. Pour tirer meilleur parti de la présence de ce repère 40, la lame 2 est par ailleurs préférentiellement positionnée entre les éléments de réception 11 du moyen de réception 10, de sorte que le bord coupant 5 de ladite lame 2 s'étende en sens opposé audit repère 40 relativement au premier axe A-A'. En d'autres termes, l'outil coupant que forme avantageusement la lame 2 s'étend ainsi selon un axe confondu avec le premier axe A-A', le repère 40 étant positionné en regard du dos de ladite lame 2.

De façon particulièrement avantageuse, ledit repère 40 est un repère 40 visuel et tactile. De la sorte, le repère 40 reste parfaitement perceptible du chirurgien en toute situation, en particulier même dans le cas où le deuxième élément 22 du dispositif de verrouillage / déverrouillage 14 est souillé, par exemple par du sang, et le repère 40 difficilement visible à l'oeil. Afin d'assurer ce caractère à la fois visuel et tactile, ledit repère 40 est, selon le mode de réalisation préférentielle illustré aux figures 1 et 5, avantageusement formé par un bourrelet 41 de matière faisant saillie radialement de ladite paroi extérieure 32 du corps principal secondaire 27 du deuxième élément 22. Selon le mode de réalisation préférentiel illustré aux figures, ce bourrelet 41 vient avantageusement de matière avec le corps principal secondaire 27 du deuxième élément 22 et constitue ainsi avec ledit corps principal secondaire 27 une pièce monobloc d'un seul tenant. Alternativement, il est toutefois envisageable que ce bourrelet 41 constitue une pièce distincte dudit corps principal secondaire 27 à laquelle il serait rapporté par exemple par collage ou encastrement.

De façon préférentielle, l'instrument chirurgicale 1 ci-dessus décrit est à usage unique, c'est-à-dire qu'il est destiné à être détruit ou recyclé / valorisé à l'issue de son utilisation pour un patient et une opération chirurgicale donnés. Cela permet non seulement une plus grande sécurité sanitaire, mais également un gain économique important pour le chirurgien et le centre de soins dans lequel il exerce, les coûts liés au nettoyage, à la stérilisation et au reconditionnement d'instruments chirurgicaux réutilisables étant particulièrement élevés en regard du coût de l'instrument en lui-même. A ce titre, ledit instrument chirurgicale 1 est avantageusement réalisé en matériau polymère, par exemple un matériau polymère composite à base de polyarylamide (PAA), ce dernier étant optionnellement chargé, comme par exemple un matériau polymère renforcé à base de polyarylamide de la gamme *« IXEF* »® commercialisée par la société SOLVAY. Un tel matériau présente en effet l'avantage d'être un matériau biocompatible doté d'une bonne résistance à la fatigue et d'une bonne ténacité. Il peut optionnellement être chargé en fibres, par exemple de carbone ou de verre, de manière à conférer à l'instrument chirurgical 1 une grande rigidité et une excellente résistance mécanique en flexion. De plus, les matériaux à base de polyarylamide sont connus pour être particulièrement faciles à mettre en oeuvre, en particulier par moulage par injection, même à haute teneur en fibres. Ils présentent un faible retrait au moulage et permettent ainsi la réalisation précise et répétable de pièces de faibles dimensions et épaisseurs et de formes complexes.

L'invention concerne en outre en tant que tel un procédé de fabrication d'un instrument chirurgical 1 tel que décrit précédemment. Selon ce procédé, on réalise par moulage ledit instrument chirurgical 1 dans son intégralité. En d'autres termes, chaque élément que comporte ledit instrument chirurgical 1, à savoir en particulier l'organe de réception 7 et les premier 15 et deuxième 22 éléments du dispositif de verrouillage / déverrouillage 14, est obtenu par le biais d'une seule opération ou de plusieurs d'opérations distinctes de moulage, par exemple par injection, et sans autre opération de finition ou de reprise du genre usinage ou autre. La matière destinée à être moulée au cours du procédé de fabrication est avantageusement un matériau polymère, préférentiellement un matériau polymère composite à base de polyarylamide (PAA) tel que mentionné ci-avant. De façon préférentielle, ledit procédé de fabrication est mis en oeuvre exclusivement à l'aide d'outils de moulage en dépouille. On entend par là que ledit procédé ne met préférentiellement en oeuvre aucun outil de moulage présentant des dépouilles négatives, ou contre-dépouilles. En particulier, ledit procédé ne met pas en oeuvre, notamment pour la réalisation du trou axial taraudé 17 du premier élément 15, de noyau fileté, couteux et complexe à extraire par rotation au démoulage.

De préférence, la fabrication de l'instrument chirurgical 1 se déroule de la manière suivante :
- on verse ou on coule ou on injecte le matériau destiné à former l'organe de réception 7 dans un premier moule, formé par exemple de deux demi-moules se refermant selon un premier plan de joint orthogonal au premier plan d'extension P1 de la fente 12,
- on verse ou on coule ou on injecte le matériau destiné à former le premier 15 élément du dispositif de verrouillage / déverrouillage 14 dans un deuxième moule, préférentiellement à tiroirs, formé par exemple de deux demi-moules se refermant selon un deuxième plan de joint contenant le premier axe longitudinal B-B', ce deuxième moule comprenant :
   - un premier tiroir cylindrique en dépouille, positionné selon le deuxième axe longitudinal B-B' confondu avec ledit deuxième plan de joint, permettant de ménager un trou axial 17 dans l'extrémité de couplage 16 dudit premier élément 15,
   - un deuxième et un troisième tiroirs de section rectangulaire et portant en l'une de leurs extrémités une empreinte de demi-taraudage, positionnés dans un plan orthogonal audit deuxième plan de joint.
- on verse ou on coule ou on injecte le matériau destiné à former le deuxième 22 élément du dispositif de verrouillage / déverrouillage 14 dans un troisième moule, formé par exemple de deux demi-moules se refermant selon un troisième plan de joint orthogonal à l'axe longitudinal C-C' selon lequel s'étend ledit deuxième élément 22.

La mise en oeuvre du procédé de l'invention pour la réalisation, en particulier, du premier élément 15 du dispositif de verrouillage / déverrouillage 14 se traduit de manière particulièrement visible par la création de deux fenêtres 42 traversantes sensiblement rectangulaires (visibles à la figure 4), décalées l'une par rapport à l'autre le long du deuxième axe longitudinal B-B', et ménagées à travers la surface du premier élément 15 selon des plans d'extension parallèles audit deuxième plan de joint. Ces fenêtres 42 donnent chacune respectivement vue sur un des demi-taraudages 18, 19 formant le taraudage du trou axial taraudé 17 du premier élément 15 du dispositif de verrouillage / déverrouillage 14.

L'invention concerne également en tant que tel un kit chirurgicale 43 destiné à la mise en oeuvre d'une technique opératoire, préférentiellement percutanée, notamment dans le cadre du traitement d'une pathologie du pied d'un patient, comprenant un instrument chirurgical 1 conforme à l'invention, et tel que décrit ci-avant, et comprenant également au moins un écarteur 43, une rugine 44 et une râpe 45.

L'écarteur 44 est un outil chirurgical, se présentant sous la forme générale d'un levier, destiné à permettre de modifier le positionnement de corps osseux d'un patient l'un par rapport à l'autre. Un tel écarteur 44 consistant classiquement en une tige se terminant par une tête plus fine et courbée de sorte à former avec la tige une forme en « Z ». Toutefois, l'écarteur 44 que comprend le kit chirurgical 43 de l'invention comporte préférentiellement un manche de préhension relié à une tête de travail recourbée pourvue d'une gorge axiale apte notamment à recevoir et à guider une broche d'ostéosynthèse, par exemple des broches de Kirschner. En outre, cette tête de travail peut avantageusement être dotée d'ailes longitudinales incurvées, de nature à offrir un appui de large et stable à l'écarteur 44 lorsque ce dernier est manoeuvré tel un levier pour modifier le positionnement desdits corps osseux.

La rugine 45 est un outil chirurgicale destiné à racler une surface osseuse et, en particulier, à séparer des os les tissus mous environnants tels que des muscles ou des tendons, afin de pouvoir travailler en contact direct de ladite surface osseuse. Une telle rugine 45 est formée d'une petite plaque supportée par un manche. Ladite plaque est pourvue d'arêtes vives et son extrémité libre, qui forme une arête perpendiculaire à l'axe principal du manche, est biseautée de manière à former un bord d'attaque spécifiquement agressif pour assurer le décollement du périoste et des tissus mous. Ladite plaque et ledit manche forment préférentiellement une pièce monobloc d'un seul tenant. Ladite rugine 45 peut toutefois alternativement être formée d'une plaque et d'un manche distincts, ladite plaque étant insérée dans le manche de manière permanente ou encore amovible.

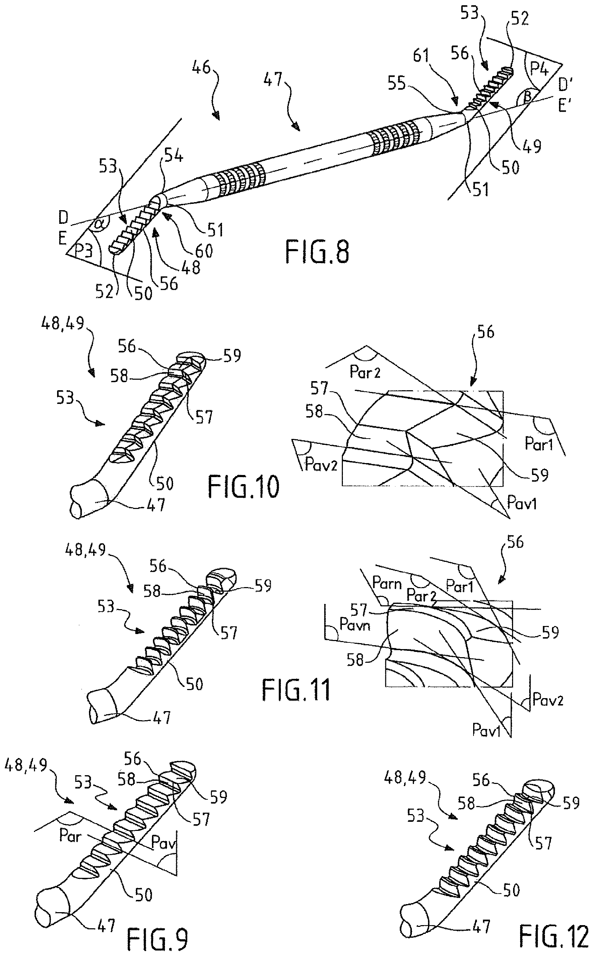

La râpe 46 (ou « *excavateur »),* dont un exemple de réalisation est illustré à la figure 8, est un outil chirurgical qui permet d'extraire du corps du patient des débris biologiques ou séquestre, constitués notamment de fragments osseux et tissulaires mélangés à du sang, en particulier ceux générés lors d'une opération d'ostéotomie, par exemple du premier métatarsien, ou de réduction du volume de la tête métatarsienne. Une telle râpe 46 comporte préférentiellement :
- d'une part, d'un manche de râpe 47 s'étendant le long d'un quatrième axe longitudinal D-D', et
- d'autre part, d'au moins une tête de râpe 48, 49, comportant un corps 50 allongé s'étendant le long d'un cinquième axe longitudinal E-E' entre une première extrémité 51 reliée au manche de râpe 47 et une deuxième extrémité 52 libre, ledit corps 50 présentant une face active 53 reliant lesdites première 51 et deuxième 52 extrémités.

Ladite au moins une tête de râpe 48, 49 s'étend avantageusement dans le prolongement du manche de râpe 47, de préférence à partir d'une des extrémités 54, 55 de ce dernier, lesdits quatrième D-D' et cinquième E-E' axes étant préférentiellement confondus.

Ladite face active 53 de la tête de râpe 48, 49 est pourvue d'une rangée de dents 56 parallèles, alignées selon le cinquième axe longitudinal E-E', et présentant des arêtes 57 vives aptes à venir râper efficacement des corps osseux et tissus mous environnants afin d'extraire le séquestre. Classiquement ces dents 56 se présentent selon une configuration « *droite* », illustrée à la figure 9, c'est-à-dire qu'elles s'étendent linéairement dans une direction orthogonale au cinquième axe longitudinal E-E', leurs faces avant 58 et arrière 29 s'inscrivant respectivement dans un seul et unique plan Pav, Par. De manière préférentielle, les faces avant 58 et arrière 59 desdites dents 56 s'inscrivent respectivement dans au moins deux plans Pav1, Pav2, Par1, Par2 sécants distincts, de sorte que lesdites dents 56 peuvent avantageusement présenter une configuration géométrique alternative parmi au moins les suivantes :
- une configuration *« en chevron » :* selon cette première variante, illustrée à la figure 10, les faces avant 58 et arrière 59 desdites dents 56 s'inscrivent respectivement dans deux plans Pav1, Pav2, Par1, Par2 sécants distincts, de sorte que lesdites dents 40 présentent un profil concave en « *V* », l'ouverture du « *V* » s'ouvrant préférentiellement en direction du manche de râpe 47,
- une première configuration « *concave » :* selon cette seconde variante, illustrée à la figure 11, les faces avant 58 et arrière 59 desdites dents 56 s'inscrivent préférentiellement respectivement dans une multitude de plans Pav1, Pav2,..., Pavn, PAr1, Par2,..., Par*n* sécants distincts, de sorte que lesdites dents 56 présentent un profil concave curviligne, la concavité des dents 56 s'ouvrant de préférence en direction du manche de râpe 47 ;
- une deuxième configuration « *concave* » : selon cette troisième variante, illustrée à la figure 12, les faces avant 58 et arrière 59 desdites dents 56 s'inscrivent préférentiellement dans une multitude de plans Pav1, Pav2,..., Pavn, PAr1, Par2,..., Par*n* sécants distincts, de sorte que lesdites dents 56 présentent un profil concave curviligne, la concavité des dents 56 s'ouvrant de préférence en direction opposée à celle du manche de râpe 47.

Il a en effet été observé que ces configurations *« en chevron »* et concaves, en comparaison avec la configuration « *droite »* classique, permettent avantageusement de bloquer dans la concavité de chaque dent davantage de séquestres osseux et ainsi de les extraire de la plaie plus efficacement et rapidement, ce qui contribue à la réduction du temps opératoire. Bien évidemment, les dents 56 de ladite tête de râpe 48,49, peuvent présenter d'autres variantes avantageuses de configuration géométrique que celles décrites ci-dessus, par exemple une configuration selon un profil en « *W ».*

Le corps 50 de ladite tête de râpe 48, 49 est préférentiellement relié en sa première extrémité 51 au manche de râpe 47 de façon ferme, ladite tête de râpe 48, 49 et ledit manche de râpe 47 étant rendu solidaires l'un de l'autre. La tête de râpe 48, 49 peut ainsi constituer une pièce indépendante du manche de râpe 47, éventuellement interchangeable, rapportée et maintenue fermement à ce dernier par exemple par collage, vissage, clipsage ou encore par enfichage. Toutefois, de manière préférentielle, ledit manche de râpe 47 et ladite tête de râpe 48, 49 constituent une pièce monobloc d'un seul tenant, c'est-à-dire qu'ils constituent avantageusement une seule et même pièce réalisée en totalité dans un matériau donné, préférentiellement un matériau polymère, un matériau polymère composite à base de polyarylamide (PAA), ce dernier étant optionnellement chargé.

De façon préférentielle, et tel qu'illustré à la figure 8, ladite râpe 46 comporte avantageusement deux têtes de râpe 48, 49, chacune reliée à l'une des extrémités 54, 55 du manche de râpe 47.

Le corps 50 des têtes de râpe 48, 49 s'étendent respectivement sensiblement le long d'un premier P3 et d'un deuxième P4 plan d'extension sécants au quatrième axe longitudinal D-D', le corps 50 desdites têtes de râpe 48, 49 et le manche de râpe 47 étant respectivement reliés par une première 60 et une deuxième 61 zone d'inflexion.

Dans une variante préférentielle illustrée à la figure 8, ladite râpe 46 comporte deux têtes de râpes 48, 49. Celles-ci s'étendent respectivement dans un troisième P3 et un quatrième P4 plans d'extension, lesdits plans P3, P4 étant parallèles et sécants au quatrième axe longitudinal D-D' de sorte à former avec ce dernier un angle élévation α, β préférentiellement compris entre 135° et 165°. En outre, dans cette variante préférentielle, lesdites têtes de râpes 48, 49 s'étendent avantageusement en sens opposé, conférant à ladite râpe une forme en « Z », de sorte à ce que le chirurgien, lorsqu'il utilise manuellement l'une des têtes de râpe 48, 49, ne soit pas gêné par la présence de l'autre tête de râpe 48, 49. D'autres variantes sont toutefois parfaitement envisageables, les troisième P3 et un quatrième P4 plans d'extension pouvant ne pas être parallèles et les angles d'élévation α, β respectivement formés par ces plans avec le quatrième axe longitudinal D-D' pouvant être identiques ou différents, tout en étant avantageusement compris entre 135° et 165°.

Pour finir, lorsque ladite râpe 46 se présente dans une variante dans laquelle elle comporte deux têtes de râpe 48, 49, les dents 56 de celles-ci peuvent présenter des configurations géométriques, telles que décrites précédemment, identiques ou bien différentes, de sorte à offrir au chirurgien une râpe 46 parfaitement adaptée à ses besoins.

Il est entendu que la râpe 46 ci-dessus décrite, prise indépendamment dudit kit chirurgical 43, peut en tant que telle constituer une invention à part entière, distincte de la présente invention.

Sans sortir du cadre de l'invention, le kit chirurgical 43 pourra également inclure d'autres outils chirurgicaux que ceux décrits ci-dessus, par exemple une ou plusieurs lame 2 de bistouri, une ou plusieurs broches d'ostéosynthèse, par exemple des broches de Kirschner, ou encore une ou plusieurs fraises percutanées permettant de réaliser des découpes osseuses.

Tel qu'illustré à la figure 7, ledit kit 43 est avantageusement constitué d'un étui 62, par exemple en plastique transparent de type polypropylène, dans lequel sont disposés lesdits instrument chirurgical 1, écarteur 44, rugine 45 et râpe 46, ces derniers étant préférentiellement calés dans ledit étui 62 par une mousse de calage.

De manière préférentielle, ledit kit chirurgical 43 est à usage unique, c'est-à-dire que lesdits instrument chirurgicale 1, écarteur 44, rugine 45 et râpe 46 qui le composent sont destinés à être détruits ou recyclés / valorisés à l'issue de leur utilisation pour un patient et une opération chirurgicale donnés. Il est cependant envisageable, sans sortir du cadre de l'invention, que ledit kit 43 soit au contraire réutilisable, c'est-dire que lesdits instrument chirurgical 1, écarteur 44, rugine 45 et râpe 46 qui le composent pourraient, après une première utilisation, être à nouveau proposés au chirurgien après avoir subi un retraitement et un reconditionnement adéquat. A ce titre, lesdits instrument chirurgical 1, écarteur44, rugine 45 et râpe 6 sont avantageusement sont tous réalisés en matériau polymère, par exemple un matériau polymère composite à base de polyarylamide (PAA), ce dernier étant optionnellement chargé. De manière encore plus préférentielle, lesdits instrument chirurgical 1, écarteur 44, rugine 45 et râpe 46 sont avantageusement conditionnés de manière stérile dans l'étui 62, de sorte à être immédiatement utilisables par le chirurgien sans étape de nettoyage et de stérilisation préalable.

En définitive, lorsqu'il est équipé d'un outil coupant, par exemple d'une lame 2 de bistouri, l'instrument chirurgical 1 de l'invention, particulièrement ergonomique et sûr d'utilisation, permet au chirurgien de réaliser une technique opératoire percutanée avec un minimum de risque d'endommagement involontaire des tissus mous environnants. Réalisé à partir d'un procédé de fabrication simple, l'instrument chirurgical 1 est relativement peu couteux à produire. Il est en outre avantageusement intégré dans le kit chirurgical 43 de l'invention, de manière à offrir au chirurgien un ensemble complet, ergonomique, sur et peu coûteux, d'outils destinés la mise en oeuvre d'une technique opératoire, préférentiellement percutanée, de traitement d'une pathologie osseuse, notamment du pied.

## Revendications

1. Instrument chirurgical (1) destiné à recevoir de manière amovible un outil coupant, par exemple une lame (2) de bistouri, comportant :
- un organe de réception (7), qui comporte lui-même un corps principal primaire (8) allongé avec une extrémité distale primaire (9), laquelle est dotée d'un moyen de réception (10) de l'outil coupant, ledit moyen de réception (10) étant conçu pour évoluer entre une configuration verrouillée et une configuration déverrouillée,
- un dispositif de verrouillage / déverrouillage (14), qui permet de faire passer le moyen de réception (10) de sa configuration verrouillée à sa configuration déverrouillée et vice versa, ledit dispositif de verrouillage / déverrouillage (14) comprenant un premier élément (15) relié à l'organe de réception (7) par une liaison hélicoïdale,
et un deuxième élément (22), comprenant un corps principal secondaire (27) présentant une paroi extérieure (32), relié à l'organe de réception (7) par une liaison glissière, pour qu'une rotation relative des premier (15) et deuxième (22) éléments entraîne une translation relative de l'organe de réception (7) et du deuxième élément (22) permettant ainsi de commander le verrouillage / déverrouillage du moyen de réception (10), **caractérisé en ce que** ledit premier élément (15) constitue un manche de préhension (36) dudit instrument chirurgical (1) et **en ce que** le dispositif de verrouillage / déverrouillage (14) est pourvu d'un repère (40) qui reflète la position de l'outil coupant relativement au deuxième élément (22), ledit repère 40 étant positionné axialement sur ladite paroi extérieure (32).

2. Instrument chirurgical (1) selon l'une quelconque des revendications précédentes, **caractérisé en ce que** le moyen de réception (10) comprend au moins deux éléments de réception (11) flexibles définissant une fente (12) axiale allongée apte à recevoir ledit outil coupant.

3. Instrument chirurgical (1) selon la revendication 2, **caractérisé en ce que** ledit corps principal primaire (8) de l'organe de réception (7) est de forme sensiblement cylindrique et présente une surface extérieure (25), laquelle surface extérieure (25) est pourvue d'au moins un méplat primaire (26) axial s'étendant :
- longitudinalement depuis ladite extrémité distale primaire (9) sur au moins une portion de la longueur dudit corps principal primaire (8) de l'organe de réception (7),
- et selon un deuxième plan d'extension (P2) orthogonal au premier plan d'extension (P1) de ladite fente (22).

4. Instrument chirurgical (1) selon la revendication précédente, **caractérisé en ce que** le corps principal secondaire (27) dudit deuxième élément 22 s'étendant entre une extrémité distale secondaire (28) et une extrémité proximale secondaire (29), ledit corps principal secondaire (27) est pourvu d'un trou axial cylindrique (30) traversant apte à recevoir étroitement ledit corps principal primaire (8) de l'organe de réception (7), ledit trou axial cylindrique (30) débouchant de ladite extrémité distale secondaire (28) à travers un orifice distal (33) et de ladite extrémité proximale secondaire (29) à travers un orifice proximal.

5. Instrument chirurgical (1) selon la revendication précédente, **caractérisé en ce que** ledit trou axial cylindrique (30) définit une paroi intérieure (34), laquelle est pourvue d'au moins un méplat secondaire (35) axial, de sorte que ce méplat secondaire (35) forme par coopération avec le méplat primaire (26) un dispositif de blocage (23) apte à interdire toute rotation d'axe (A-A') dudit organe de réception (7) dans ledit deuxième élément (22) du dispositif de verrouillage / déverrouillage (14).

6. Instrument chirurgical (1) selon les revendications 2 à 5 **caractérisé en ce que** :
- ladite surface extérieure (25) du corps principal primaire (8) de l'organe de réception (7) est pourvue de deux méplats primaires (26) axiaux, et
- ladite paroi intérieure (34) est pourvue de deux méplats secondaires (35) axiaux,
lesdits méplats primaires (26) axiaux et méplats secondaires (35) axiaux étant respectivement positionnés en opposition radiale l'un par rapport à l'autre, de sorte lesdits méplats secondaires (35) forment respectivement par coopération avec lesdits méplats primaires (26) un double un dispositif de blocage (23) apte à interdire toute rotation d'axe A-A' dudit organe de réception (7) dans ledit deuxième élément (22) du dispositif de verrouillage / déverrouillage (14).

7. Instrument chirurgical (1) selon l'une quelconque des revendications précédentes, **caractérisé en ce que** ledit corps principal secondaire (27) du deuxième élément (22) s'étend entre une extrémité distale secondaire (28) et une extrémité proximale secondaire (29), ledit repère (40) s'étendant longitudinalement à partir de ladite extrémité distale secondaire (28) sur au moins une partie, et préférentiellement sur la totalité, de la longueur du corps principal secondaire (27) dudit deuxième élément (22).

8. Instrument chirurgical (1) selon l'une quelconque des revendications précédente, **caractérisé en ce que** ledit repère (40) est un repère (40) visuel et tactile.

9. Instrument chirurgical (1) selon la revendication précédente, caractérisé en ce ledit repère (40) est formé par un bourrelet (41) de matière faisant saillie radialement de ladite paroi extérieure (32) du corps principal secondaire (27) du deuxième élément (22).

10. Instrument chirurgical (1) selon la revendication précédente, **caractérisé en ce que** l'organe de réception (7) est prolongé axialement à partir d'une extrémité proximale primaire (20) par une barre filetée (21) et **en ce que** ledit premier élément (15) comporte une extrémité de couplage (16) pourvue d'un trou axial taraudé (17) dont le taraudage est préférentiellement constitué de deux demi-taraudages (18,19) positionnés de manière décalée l'un par rapport à l'autre.

11. Instrument chirurgical (1) selon l'une quelconque des revendications précédentes, **caractérisé en ce qu'**il est à usage unique.

12. Instrument chirurgical (1) selon l'une quelconque des revendications précédentes, **caractérisé en ce qu'**il est réalisé en matériau polymère, par exemple polyéther-éther-cétone PEEK, ce dernier étant optionnellement chargé.

13. Procédé de fabrication d'un instrument chirurgical (1) selon l'une quelconque des revendications précédentes, **caractérisé en ce qu'**on réalise par moulage ledit instrument chirurgical (1) dans son intégralité, de préférence exclusivement à l'aide d'outils de moulage en dépouille.

14. Kit chirurgical (43) destiné à la mise en oeuvre d'une technique opératoire, préférentiellement percutanée, notamment dans le cadre du traitement d'une pathologie du pied d'un patient, comprenant un instrument chirurgical 1 selon l'une quelconque des revendications 1 à 19, et étant caractérisé en ce qui comprend également au moins un écarteur 43, une rugine 44 et une râpe 45, ledit kit chirurgical (43) étant de préférence à usage unique.

15. Kit chirurgical (43) selon la revendication précédente, **caractérisé en ce que** lesdits instrument chirurgical (1), écarteur (44), rugine (45) et râpe (46) sont tous réalisés par moulage d'un matériau polymère, par exemple du polyéther-éther-cétone PEEK, ce dernier étant optionnellement chargé en carbone.
